# EUROPÄISCHE PATENTSCHRIFT

(11) **EP 1 737 833 B1**
(45) Veröffentlichungstag und Bekanntmachung des Hinweises auf die Patenterteilung: **05.08.2009**
(21) Anmeldenummer: 05739539.4
(22) Anmeldetag: 12.04.2005
(51) Int. Cl.: C07D 251/60, C07D 251/62

(54) **VERFAHREN ZUR HERSTELLUNG VON REINEM MELAMIN**
METHOD FOR PRODUCING PURE MELAMINE
PROCEDE DE PRODUCTION DE MELAMINE PURE

(30) Priorität: 14.04.2004 DE 102004018784
(43) Veröffentlichungstag der Anmeldung: 03.01.2007
(73) Patentinhaber: AMI Agrolinz Melamine International GmbH, 4021 Linz (AT)
(72) Erfinder: RUECH, Wolfgang, A-4753 Taiskirchen (AT)
(74) Vertreter: Gross, Felix
(86) Internationale Anmeldenummer: PCT/EP2005/003994
(87) Internationale Veröffentlichungsnummer: WO 2005/100327

(56) Entgegenhaltungen:
- WO-A-00/29393
- WO-A-03/045927
- US-A- 3 132 143

## Beschreibung

Die Anmeldung betrifft ein Verfahren zur Herstellung von reinem Melamin gemäß Anspruch 1.

Bei den Hochdruckverfahren zur Herstellung von Melamin werden im allgemeinen Harnstoffschmelze und gegebenenfalls gasförmiges Ammoniak ohne Anwesenheit eines Katalysators etwa bei Temperaturen zwischen 325 und 450 °C, bevorzugt zwischen 350 und 425 °C und Drücken zwischen 50 und 250 bar zu flüssigem Melamin und Offgas umgesetzt. Das Reaktionsoffgas besteht hauptsächlich aus Ammoniak und Kohlendioxid, mit geringen Mengen an gasförmigem Melamin. Das flüssige Rohmelamin enthält neben nicht umgesetztem Harnstoff auch Nebenprodukte wie beispielsweise Melem, Melam oder weitere Kondensationsprodukte des Melamins, welche im Endprodukt unerwünscht sind und deshalb abgetrennt werden müssen. Für die Abtrennung der Melaminnebenprodukte vom Melamin wird die bekannte Tatsache ausgenutzt, dass die Nebenprodukte mit Wasser, vorzugsweise in Gegenwart von Alkalien zu Oxoaminotriazinverbindungen wie Ammelin und Ammelid hydrolysieren. Während der nachfolgenden Melaminkristallisation werden diese in Lösung gehalten, sodass das reine Melamin selektiv auskristallisiert. Bei diesen Melaminverfahren wird die Melaminschmelze aus dem Hochdruckteil in einem anschließenden Niederdruckteil in Gegenwart von Wasser aufgearbeitet.

Gemäß US 3 132 143 beispielsweise wird die Reaktionsmischung aus dem Hochdruck-Synthesereaktor, bestehend aus der Melaminschmelze und dem Offgas einem Quencher zugeführt, in welchem die Mischung mit einer wässrigen, an Ammoniak und Kohlendioxid gesättigten Lösung bei 100 bis 200 °C und 10 bis 35 bar während 10 bis 60 min kontaktiert wird. Beim Kontakt mit der kühlen Quenchlösung wird das Melamin darin absorbiert, während der Großteil des Offgases abgetrennt wird. Zum Abbau der Nebenprodukte wird die Melaminlösung während 20 bis 50 min verweilen gelassen, anschließend das enthaltene NH₃ und CO₂ mit Hilfe von Dampf entfernt und nach Zugabe von alkalihältiger Mutterlauge und Filtration unlöslicher Produkte das Melamin auskristallisiert.

Nachteilig bei diesem Verfahren ist die Tatsache, dass das Melaminoffgas erst im Quencher vom Melamin abgetrennt und damit auf einem niedrigen Druckniveau und nicht wasserfrei erhalten wird. Da das Offgas hauptsächlich aus NH₃ und CO₂ besteht, werden beträchtliche CO₂ Mengen in den Nassteil der Anlage eingetragen. Über die CO₂-hältige Quenchflüssigkeit wird bereits abgetrenntes CO₂ sogar erneut in den Melaminprozess rezykliert, wodurch der CO₂ Gehalt der Melaminlösung erhöht und somit der pH Wert der Lösung erniedrigt wird. Dies ist insbesondere für den bevorzugt im alkalischen Bereich ablaufenden Nebenproduktabbau während des Verweilens im Quencher von Nachteil, da dieser unter diesen Bedingungen langsam und unvollständig verläuft. Das gesamte CO₂ wird erst im Dampfstripper des Nassteils aus der Melaminlösung abgestrippt. Dies ist energetisch zudem sehr aufwendig.

Gemäß WO 00/29393 A1 oder WO 03/045927 A1 werden Melaminherstellungsverfahren beschrieben, bei welchen bereits im Hochdruckreaktor die Melaminschmelze vom Reaktionsoffgas separiert wird. Das Offgas wird wasserfrei und mit hohem Druck erhalten und in die Harnstoffanlage rückgeführt. Die der weiteren Aufarbeitung zugeführte Melaminschmelze enthält demnach bereits einen um den Offgasanteil verminderten CO₂-Gehalt. In weiterer Folge wird das in der Melaminschmelze gelöste CO₂, beispielsweise durch Hindurchleiten von NH₃ aus der Schmelze entfernt. Die so vorbehandelte Melaminschmelze wird anschließend dem Quencher zugeführt, in welchem durch Kontakt mit einer wässrigen, alkalihältigen Lösung die Melaminschmelze in eine Melaminsuspension bzw. -lösung überführt wird. Um den Nebenproduktabbau zu beschleunigen und die dabei entstehenden Oxoaminotriazinverbindungen Ammelin und Ammelid in Lösung zu halten, wird der Melaminlösung NaOH zugesetzt, bevor sie zum Nebenproduktabbau verweilen gelassen wird. Anschließend wird noch vorhandenes gelöstes NH₃ ausgestrippt und das Melamin schließlich auskristallisiert.

Nachteilig bei diesen Verfahren ist die Tatsache, dass in der vom Quencher ausgetragenen Melaminsuspension bzw. -lösung noch immer beträchtliche Mengen an CO₂ vorhanden sind. Diese ergeben sich aufgrund unvollständiger CO₂-Entfernung im Hochdruckteil und durch hydrolytische Zersetzung von nicht umgesetztem Harnstoff sowie Melaminnebenprodukten im Quencher. Das gesamte vorhandene CO₂ muss durch Zugabe von NaOH neutralisiert werden. Erst bei weiterer NaOH-Zugabe kommt es zum Ansteigen des pH-Wertes. Ein hoher pH-Wert wird für einen raschen Nebenproduktabbau beim nachfolgenden Verweilen der Melaminlösung benötigt. Dadurch ergeben sich für die gewünschte hohe Melaminreinheit mit niedrigen Nebenproduktgehalten sehr hohe NaOH-Mengen, welche aus wirtschaftlichen und logistischen Gründen unerwünscht sind.

Es stellte sich demnach die Aufgabe, ein Melaminverfahren zu finden, das bei energetisch gleichbleibenden Anlagenkennzahlen und gleichbleibender Qualität des Endproduktes Melamin einen verminderten NaOH-Verbrauch aufweist.

Erfindungsgemäß wird dieses Ziel dadurch erreicht, dass die Melaminschmelze mit Wasser mit einer Reinheit von mehr als 95 Gew% gequencht wird und dass das in der Melaminlösung vorhandene CO₂ und NH₃ vor der NaOH-Zugabe und dem Verweilenlassen zum Nebenproduktabbau entfernt wird.

Gegenstand der vorliegenden Erfindung ist demnach ein Verfahren zur Herstellung von reinem Melamin durch Aufarbeitung einer aus einem Hochdruckverfahren erhaltenen und von den Reaktionsoffgasen befreiten Melaminschmelze, bei dem
a) die Melaminschmelze mit Wasser mit einer Reinheit von mehr als 95 Gew% gequencht wird, anschließend
b) aus der erhaltenen Melaminlösung NH₃ und CO₂ entfernt werden und dann
c) die Melaminlösung mit Alkali versetzt und anschließend verweilen gelassen wird,
d) worauf durch Kristallisation reines Melamin erhalten wird.

Erfindungsgemäß wird direkt nach dem Quenchen mit Wasser mit einer Reinheit von 95 Gew% das gesamte, einerseits aus dem Hochdruckteil mitgeschleppte und andererseits im Quencher selbst durch Hydrolyse gebildete CO₂ aus der Melaminlösung entfernt. Somit wird anschließend eine nahezu CO₂-freie Melaminlösung mit Alkali versetzt, was zu einem sofortigen pH-Wert-Anstieg in der Melaminlösung führt. Da ein hoher pH-Wert für einen raschen Nebenproduktabbau beim nachfolgenden Verweilenlassen benötigt wird, wird beim erfindungsgemäßen Verfahren mit geringeren Alkalimengen dieselbe Melaminqualität wie bei den bekannten Vergleichsverfahren erzielt.

Im vorliegenden Verfahren kann jede aus einem Hochdruckverfahren stammende Melaminschmelze nach Abtrennung der Reaktionsoffgase verwendet werden. Besonders reines Melamin wird erhalten, wenn die Melaminschmelze vor dem Quenchen, im Hochdruckteil der Melaminanlage, vorgereinigt wird. Beispielsweise ist es möglich, in der Schmelze vorhandene Nebenprodukte durch Kühlen und / oder Verweilen der Melaminschmelze unter hohem Ammoniakdruck teilweise zu entfernen. Es ist vorteilhaft, im vorliegenden Verfahren ammoniakgesättigte Melaminschmelze zu verwenden.

In einer bevorzugten Ausführungsform wird bereits vor dem Quenchen das in der Melaminschmelze gelöste CO₂ weitgehend entfernt. Dies geschieht beispielsweise durch Behandeln der Melaminschmelze mit gasförmigem Ammoniak. Auf diese Weise müssen im Nassteil nach dem Quenchen nur mehr geringere CO₂-Anteile aus der Melaminlösung entfernt werden, was energetisch von Vorteil ist.

Die nach dem vorliegenden Verfahren aufzuarbeitende Melaminschmelze wird mit einer Temperatur von etwa 330 bis 400 °C, bevorzugt von etwa 330 bis 380 °C, besonders bevorzugt von etwa 330 bis 360 °C und einem Druck von etwa 50 bis 600 bar, bevorzugt von etwa 50 bis 250 bar, besonders bevorzugt von etwa 70 bis 170 bar dem Quencher zugeführt.

Im Quencher erfolgt das Abschrecken der Melaminschmelze mit Wasser mit einer Reinheit von mehr als 95 Gew%, wodurch die Melaminschmelze in eine Melaminlösung überführt wird.

Vorteilhaftereise wird als Wasser Kesselspeisewasser und / oder Dampfkondensat verwendet. Weiters ist es möglich, das aufgearbeitete und gereinigte Abwasser der Melaminanlage zum Abschrecken der Melaminschmelze im Quencher zu verwenden.

Vorteilhafterweise erfolgt das Quenchen der Melaminschmelze bei 170 bis 220 °C, besonders bevorzugt bei 180 bis 200 °C. Die erhöhte Temperatur beim Quenchen ermöglicht es, dass durch die größere Melaminlöslichkeit eine höher konzentrierte Melaminlösung erhalten wird. Dies ermöglicht kleinere Apparatevolumina im Nassteil und bringt energetische Vorteile beim Abtrennen von NH₃und CO₂ und durch das Rezyklieren geringerer Wassermengen. Nicht umgesetzter Harnstoff oder auch Zwischenprodukte werden im Quencher zu NH₃ und CO₂ hydrolysiert. Die genaue Temperatur im Quencher ist über das Mengenverhältnis von Quenchwasser zu Melaminschmelze und/oder über die Quenchwassertemperatur einstellbar. Der Druck im Quencher ist beispielsweise der sich bei der jeweiligen Temperatur einstellende Gleichgewichtsdruck.

Es ist vorteilhaft, wenn die beim Quenchen erhaltene Melaminlösung eine Melaminkonzentration von 10 bis 40 Gew%, bevorzugt von 20 bis 30 Gew%, besonders bevorzugt von 25 Gew% aufweist. In diesem Fall beträgt das Verhältnis Quenchwasser zu Melaminschmelze etwa 3 t Quenchwasser / t Melaminschmelze.
Aus der aus dem Quencher ausgetragenen Melaminlösung werden anschließend NH₃ und CO₂ entfernt. Dies erfolgt vorteilhafterweise bei annähernd derselben oder bei einer höheren Temperatur wie das Quenchen. Dadurch wird erreicht, dass die im Quencher beginnende Hydrolyse fortgesetzt und das entstehende CO₂ und NH₃ sofort abgetrennt werden kann. Die Temperatur wird so hoch gewählt, dass beim Entfernen von NH₃ und CO₂ keine Gefahr hinsichtlich Auskristallisieren von Melamin besteht. Nach der CO₂- und NH₃-Entfernung wird eine gereinigte Melaminlösung mit etwa derselben Melaminkonzentration wie am Quencheraustrag erhalten. Beispielsweise beträgt die Melaminkonzentration etwa 25 Gew% und die Temperatur etwa 200 °C.

Von Vorteil ist es, wenn die Entfernung von NH₃ und CO₂ aus der Melaminlösung in einer Rektifikationskolonne erfolgt, wobei NH₃ und CO₂ aus der Melaminlösung mit Dampf ausgestrippt und in Form einer möglichst konzentrierten Flüssigkeit zurückgewonnen werden. Dies hat den Vorteil, dass bei einer Rückführung der zurückgewonnenen Inhaltsstoffe in eine Harnstoffanlage oder in den Flüssigdüngerbereich der Energieeinsatz in der jeweiligen Anlage nicht durch zu große Wasserzufuhr verschlechtert wird. Die Rückgewinnung erfolgt beispielsweise als NH₃ flüssig mit bis zu etwa 20 Gew% CO₂ oder als Ammonkarbonatlauge oder auch in Form von zwei Fraktionen als Ammonkarbonatlauge und als NH₃ flüssig.

Vor dem Verweilen der gereinigten Melaminlösung zum Nebenproduktabbau wird die Melaminlösung mit Alkali versetzt. Dabei kann als Alkali beispielsweise NaOH oder KOH eingesetzt werden. Bevorzugt wird NaOH, beispielsweise eine wässrige NaOH-Lösung mit einer NaOH-Konzentration von etwa 50 Gew% verwendet. Die Menge an Alkali beträgt etwa 30 bis 60 kg, bevorzugt 40 bis 50 kg 50%-ige NaOH pro t Melamin. Durch die Alkalizugabe kommt es zu einer Erhöhung des pH-Wertes, wobei ein pH-Bereich zwischen pH 9 und 12 vorteilhaft ist. Ein hoher pH-Wert ist für einen ausreichend raschen Nebenproduktabbau wünschenswert.

Vorteilhafterweise wird vor dem Verweilen die gereinigte, nahezu NH₃- und CO₂-freie Melaminlösung, welche eine Melaminkonzentration von ca. 10 bis 40 Gew%, bevorzugt von 20 bis 30 Gew%, besonders bevorzugt von 25 Gew% aufweist, auf eine Melaminkonzentration von 5 -20 Gew%, bevorzugt von etwa 8 Gew% verdünnt. Die Temperatur der Melaminlösung wird dabei, ausgehend von 170 bis 220 °C, bevorzugt von 180 bis 200 °C, auf 120 bis 200 °C, bevorzugt auf 125 bis 170 °C, besonders bevorzugt auf 130 °C, abgekühlt. Durch das Verdünnen und Abkühlen der Melaminlösung ergibt sich eine einfachere Betriebsweise für die nachfolgenden Melaminaufarbeitungsschritte.

Von Vorteil ist es, wenn das Verdünnen und Abkühlen durch Versetzen mit einer rückgeführte Kristallisationsmutterlauge enthaltenden Lösung erfolgt. Da die Kristallisationsmutterlauge alkalihältig ist, kann auf diese Weise die Frischalkalizufuhr verringert werden. Darüber hinaus steigt durch die Rezyklierung die Melaminausbeute der Anlage und die aufzuarbeitende Abwassermenge sinkt.

Es ist möglich, die Alkalizugabe und die Zugabe der Verdünnungs- und Abkühlungslösung gleichzeitig durchzuführen. Beispielsweise können Alkali sowie Verdünnungs-und Abkühlungslösung gemischt und anschließend gemeinsam der Melaminlösung zugeführt werden. Dabei wird der Vorteil einer guten Durchmischung und homogenen Verteilung der einzelnen Ströme erzielt. Weiters ist es möglich, dass die Alkalizugabe und die Zugabe der Verdünnungs- und Abkühlungslösung getrennt voneinander in beliebiger Reihenfolge erfolgen.

Im Anschluß an die Alkalizugabe erfolgt ein Verweilen der Melaminlösung. Dabei werden Nebenprodukte wie beispielsweise Melem oder Melam abgebaut. Die Verweildauer beträgt vorteilhafterweise 5 bis 60, bevorzugt 20 bis 40 min. Auf diese Weise kann die parallel zum Nebenproduktabbau stattfindende unerwünschte Melaminhydrolyse niedrig gehalten werden.

Nach dem Nebenproduktabbau wird aus der Melaminlösung, gegebenenfalls nach einer pH-Werteinstellung das Melamin auskristallisiert. Dies erfolgt beispielsweise durch Temperaturerniedrigung und/oder Anlegen eines Vakuums. Nach anschließender Filtration und Trocknung wird reines Melamin erhalten.

Das mit dem vorliegenden Verfahren erhältliche Melamin weist eine Reinheit von mindestens 99,8 % auf und kann jeder gewünschten Weiterverarbeitung zugeführt werden.

### Beispiel 1:

### NaOH-Verbrauch in einem Melaminverfahren gemäß dem Stand der Technik

Die im Reaktor erzeugte Rohmelaminschmelze wird von den Reaktionsoffgasen getrennt, die Melaminschmelze anschließend durch Hindurchleiten von NH₃ behandelt und daraufhin in einen Quencher eingetragen. Da die Melaminschmelze 1,5 Gew.% an CO₂ enthält, werden 15 kg CO₂ pro t Melaminschmelze in den Quencher eingetragen.
Im Quencher wird die Melaminschmelze mit NaOH-hältiger Flüssigkeit kontaktiert. Gemäß der Reaktionsgleichung

2 NaOH + CO₂ → Na₂CO₃ + H₂O

reagiert die NaOH mit dem vorhandenen CO₂ zu Na₂CO₃ und steht somit nicht für die zum Nebenproduktabbau erwünschte pH-Wert-Erhöhung zur Verfügung.

Da pro mol CO₂ 2 Mole NaOH benötigt werden, ergibt sich pro t Melaminschmelze aus dem Syntheseteil ein Bedarf von 54,5 kg 50%-iger NaOH allein für die Vernichtung des mit der Melaminschmelze in den Quencher eingetragenen CO₂.

Weitere 45,5 kg 50%-ige NaOH / t Melaminschmelze werden für den Nebenproduktabbau, das heißt, die alkalische Nebenprodukthydrolyse bis zum Erreichen der gewünschten Melaminreinheit benötigt.

Demnach ergibt sich ein Gesamt-NaOH-Verbrauch von 100 kg/t Melaminschmelze.

### Beispiel 2:

### NaOH Verbrauch in erfindungsgemäßem Verfahren

Eine aus einem Hochdruckverfahren stammende Melaminschmelze, die von den Reaktionsoffgasen befreit ist, wird vor dem Quenchen mit gasförmigen Ammoniak behandelt, um gelöstes CO₂ weitgehend aus der Melaminschmelze zu entfernen. Die so behandelte Melaminschmelze wird mit einer Temperatur von 350°C und einem Druck von 150 bar dem Quencher zugeführt. Das Quenchen erfolgt bei 200°C mit Kesselspeisewasser. Pro t Melaminschmelze werden 3 t Quenchwasser zugesetzt, was eine Melaminlösung mit einer Konzentration von 25 Gew% Melamin ergibt. Im Anschluss werden NH₃ und CO₂ aus der Melaminlösung mit Wasserdampf bei 200°C gestrippt. Die 25 Gew% Melamin enthaltende Lösung wird mit rückgeführter Kristallisationsmutterlauge auf eine Melaminkonzentration von 8 Gew% verdünnt, wobei die Temperatur der Lösung auf 130°C abgekühlt wird. 45,5 kg 50%-ige NaOH Lösung pro t zugeführter Melaminschmelze werden zum Nebenproduktabbau der Melaminlösung zugesetzt und die Lösung 30 min veweilengelassen. Im Anschluss wird das Melamin durch Temperaturerniedrigung auskristallisiert, das kristalline Melamin gefiltert und getrocknet.

Da im erfindungsgemäßen Verfahren die NaOH-Zufuhr erst nach der vollständigen Entfernung von CO₂ erfolgt, steht die gesamte zugeführte NaOH-Menge allein für den Nebenproduktabbau zur Verfügung. Demnach werden pro t Melaminschmelze 54,5kg 50%-iger NaOH eingespart, die im Vergleichsverfahren für die CO₂-Neutralisation benötigt werden. Im erfindungsgemäßen Melaminverfahren wird deshalb bei gleicher Melaminqualität eine NaOH-Ersparnis von zumindest der Hälfte gegenüber einem bekannten Vergleichsverfahren erzielt.

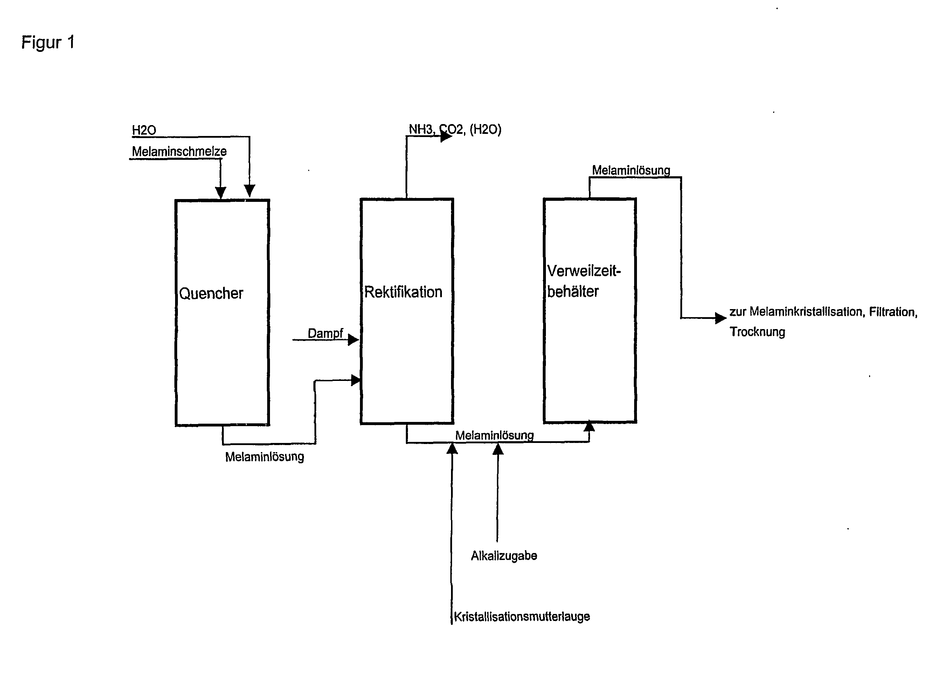

In Fig. 1 ist beispielhaft eine Ausführungsform des erfindungsgemäßen Verfahrens beschrieben.

Die aus einem Hochdruckverfahren stammende Melaminschmelze, die von den Reaktionsoffgasen befreit ist und vor dem Quenchen mit gasförmigen Ammoniak behandelt wurde, wird in einen Quencher eingeführt. Im Anschluss werden NH₃ und CO₂ aus der Melaminlösung in einer Rektifikationskolonne gestrippt. Das ausgestrippte NH₃ und CO₂ wird zur weiteren Verwendung abgeführt. Die melaminhältige Lösung wird im Anschluss mit Kristallisationsmutterlauge verdünnt. Eine alkalihaltige Lösung wird zum Nebenproduktabbau der verdünnten Melaminlösung zugesetzt und die Lösung in einem Veweilzeitbehälter ruhengelassen. Anschliessend werden die Nebeprodukte abgetrennt, das Melamin auskristallisiert, gefiltert und getrocknet.

## Patentansprüche

1. Verfahren zur Herstellung von reinem Melamin durch Aufarbeitung einer aus einem Hochdruckverfahren erhaltenen und von den Reaktionsoffgasen befreiten Melaminschmelze,
**dadurch gekennzeichnet, dass**
a) die Melaminschmelze mit Wasser mit einer Reinheit von mehr als 95Gew-% gequencht wird, anschließend
b) aus der erhaltenen Melaminlösung NH₃ und CO₂ entfernt werden und dann
c) die Melaminlösung mit Alkali versetzt und anschließend verweilen gelassen wird,
d) worauf durch Kristallisation reines Melamin erhalten wird.

2. Verfahren nach Anspruch 1, **dadurch gekennzeichnet, dass** als Wasser Kesselspeisewasser, Dampfkondensat und / oder das aufgearbeitete und gereinigte Abwasser der Melaminanlage verwendet wird.

3. Verfahren nach Anspruch 1 oder 2, **dadurch gekennzeichnet, dass** vor dem Quenchen aus der Melaminschmelze das darin gelöste CO₂, entfernt wird.

4. Verfahren nach mindestens einem der vorhergehenden Ansprüche, **dadurch gekennzeichnet, dass** das Quenchen bei 170 bis 220 °C, bevorzugt bei 180 bis 200 °C erfolgt.

5. Verfahren nach mindestens einem der vorhergehenden Ansprüche, **dadurch gekennzeichnet, dass** die nach dem Quenchen erhaltene Melaminlösung eine Melaminkonzentration von 10 bis 40 Gew-%, bevorzugt von 20 bis 30 Gew-% aufweist.

6. Verfahren nach mindestens einem der vorher genannten Ansprüche, **dadurch gekennzeichnet, dass** die Entfernung von NH₃ und CO₂ bei annähernd der gleichen oder einer höheren Temperatur wie das Quenchen erfolgt.

7. Verfahren nach mindestens einem der vorher genannten Ansprüche, **dadurch gekennzeichnet, dass** die Entfernung von NH₃ und CO₂ in einer Rektifikationskolonne erfolgt, wobei NH₃ und CO₂ mit Dampf ausgestrippt und als konzentrierte Flüssigkeit rückgewonnen wird.

8. Verfahren nach mindestens einem der vorher genannten Ansprüche, **dadurch gekennzeichnet, dass** die Rückgewinnung von NH₃ und CO₂ als NH₃ flüssig mit bis zu etwa 20 Gew% CO₂ oder als Ammonkarbonatlauge oder in Form von zwei Fraktionen als Ammonkarbonatlauge und als NH₃ flüssig erfolgt.

9. Verfahren nach mindestens einem der vorher genannten Ansprüche, **dadurch gekennzeichnet, dass** als Alkali NaOH verwendet wird.

10. Verfahren nach mindestens einem der vorher genannten Ansprüche, **dadurch gekennzeichnet, dass** die Menge an Alkali 30 bis 60 kg, bevorzugt 40 bis 50 kg an 50%-iger NaOH pro Tonne Melamin beträgt.

11. Verfahren nach mindestens einem der vorher genannten Ansprüche, **dadurch gekennzeichnet, dass** vor dem Verweilen die Melaminlösung auf eine Melaminkonzentration von 5 -20 Gew-%, bevorzugt von etwa 8 Gew-% verdünnt und auf eine Temperatur von 120 bis 200 °C, bevorzugt auf 125 bis 170 °C, besonders bevorzugt auf 130 °C, abgekühlt wird.

12. Verfahren nach mindestens einem der vorher genannten Ansprüche, **dadurch gekennzeichnet, dass** das Verdünnen und Abkühlen durch Versetzen mit einer rückgeführte Kristallisationsmutterlauge enthaltenden Lösung erfolgt.

13. Verfahren nach mindestens einem der vorher genannten Ansprüche, **dadurch gekennzeichnet, dass** das Verweilen für 5 bis 60, bevorzugt 20 bis 40 min erfolgt.

## Claims

1. Process for the preparation of pure melamine by working up a melamine melt obtained from a high pressure process and freed from the reaction off gases,
**characterized in that**
a) the melamine melt is quenched with water having a purity of more than 95% by weight,
b) NH₃ and CO₂ are then removed from the melamine solution obtained and
c) alkali is then added to the melamine solution and said solution is then allowed to reside,
d) whereupon pure melamine is obtained by crystallization.

2. Process according to Claim 1, **characterized in that** the water used is boiler feed water, condensed steam and/or the worked-up and purified waste water of the melamine plant.

3. Process according to Claim 1 or 2, **characterized in that** the CO₂ dissolved in the melamine melt is removed therefrom prior to quenching.

4. Process according to at least one of the preceding claims, **characterized in that** the quenching is effected at from 170 to 220°C, preferably at from 180 to 200°C.

5. Process according to at least one of the preceding claims, **characterized in that** the melamine solution obtained after the quenching has a melamine concentration of from 10 to 40% by weight, preferably from 20 to 30% by weight.

6. Process according to at least one of the abovementioned claims, **characterized in that** the removal of NH₃ and CO₂ is effected at virtually the same temperature as the quenching or a higher temperature than the quenching.

7. Process according to at least one of the abovementioned claims, **characterized in that** the removal of NH₃ and CO₂ is effected in a rectification column, NH₃ and CO₂ being stripped off with steam and recovered as concentrated liquid.

8. Process according to at least one of the abovementioned claims, **characterized in that** the recovery of NH₃ and CO₂ is effected as liquid NH₃ with up to about 20% by weight of CO₂ or as ammonium carbonate liquor or in the form of two fractions as ammonium carbonate liquor and as liquid NH₃.

9. Process according to at least one of the abovementioned claims, **characterized in that** the alkali used is NaOH.

10. Process according to at least one of the abovementioned claims, **characterized in that** the amount of alkali is from 30 to 60 kg, preferably from 40 to 50 kg, of 50% strength NaOH per metric ton of melamine.

11. Process according to at least one of the abovementioned claims, **characterized in that,** prior to the residence, the melamine solution is diluted to a melamine concentration of 5-20% by weight, preferably about 8% by weight, and cooled to a temperature of from 120 to 200°C, preferably to 125 to 170°C, particularly preferably to 130°C.

12. Process according to at least one of the abovementioned claims, **characterized in that** the dilution and cooling are effected by adding a solution containing recycled crystallization mother liquor.

13. Process according to at least one of the abovementioned claims, **characterized in that** the residence is effected for from 5 to 60, preferably from 20 to 40, min.

## Revendications

1. Procédé pour la préparation de mélamine pure par remise à neuf d'une fonte de mélamine qui est obtenue d'un procédé en haute pression et est défaite de gaz résiduel,
**caractérisé en ce que**
a) la fonte de mélamine est refroidie avec de l'eau étant une pureté de plus que 95 % en poids, ensuite
b) NH₃ et CO₂ sont éliminés de la solution de mélamine obtenue et puis
c) la solution de mélamine est additionnée d'alcali et ensuite est laissée séjourner,
d) où de la mélamine pure est obtenue par cristallisation.

2. Procédé selon la revendication 1, **caractérisé en ce que** de l'eau alimentée en chaudière, du condensé de vapeur et/ou de l'eau résiduelle remise en état et nettoyée de l'unité de mélamine est utilisée en tant que l'eau.

3. Procédé selon la revendication 1 ou 2, **caractérisé en ce que** le CO₂ dissous dans la fonte de mélamine est éliminé de celle-là avant le refroidissement.

4. Procédé selon au moins une des revendications précédentes, **caractérisé en ce que** le refroidissement s'effectue à 170 à 220 °C, préférentiellement à 180 à 200 °C.

5. Procédé selon au moins l'une des revendications précédentes, **caractérisé en ce que** la fonte de mélamine obtenue après le refroidissement présent une concentration de mélamine de 10 à 40 % en poids, préférentiellement de 20 à 30 % en poids.

6. Procédé selon au moins l'une des revendications précitées, **caractérisé en ce que** l'élimination du NH₃ et CO₂ s'effectue à une température approximative pareille à ou plus élevée que la température du refroidissement.

7. Procédé selon au moins l'une des revendications précitées, **caractérisé en ce que** l'élimination du NH₃ et CO₂ s'effectue dans une colonne de rectification, NH₃ et CO₂ étant éliminés avec du vapeur et étant recouvrés comme liquide concentré.

8. Procédé selon au moins l'une des revendications précitées, **caractérisé en ce que** le recouvrage du NH₃ et CO₂ s'effectue comme NH₃ liquide avec du jusqu'à environ 20 % en poids du CO₂ ou comme une base de carbonate d'ammonium ou dans la forme de deux fractions comme une base de carbonate d'ammonium et comme NH₃ liquide.

9. Procédé selon au moins l'une des revendications précitées, **caractérisé en ce que** NaOH est utilisé comme alcali.

10. Procédé selon au moins l'une des revendications précitées, **caractérisé en ce que** le montant d'alcali est de 30 à 60 kg, préférentiellement de 40 à 50 kg de NaOH étant une concentration en pourcentage de 50 par tonne de mélamine.

11. Procédé selon au moins l'une des revendications précitées, **caractérisé en ce que** la solution de mélamine est diluée à une concentration de 5-20 % en poids, préférentiellement de environ 8 % en poids, et est rafraîchie à une température de 120 à 200 °C, préférentiellement à 125 à 170 °C, particulièrement préférentiellement à 130 °C, avant le séjour.

12. Procédé selon au moins l'une des revendications précitées, **caractérisé en ce que** la dilution et le rafraîchissement s'effectuent par mélanger avec une solution contenant liqueur mère de cristallisation retourné.

13. Procédé selon au moins l'une des revendications précitées, **caractérisé en ce que** le séjour s'effectue pendant 5 à 60, préférentiellement 20 à 40 min.
